# EUROPEAN PATENT SPECIFICATION

(11) **EP 4 685 481 B1**
(45) Date of publication and mention of the grant of the patent: **09.12.2026**
(21) Application number: 25184466.8
(22) Date of filing: 23.06.2025
(51) Int. Cl.: G01N 33/18

(54) **METHOD AND SYSTEM FOR PREDICTING GROWTH OF COLIFORM BACTERIA IN ENVIRONMENTS**
VERFAHREN UND SYSTEM ZUR VORHERSAGE DES WACHSTUMS VON COLIFORMEN BAKTERIEN IN UMGEBUNGEN
PROCÉDÉ ET SYSTÈME DE PRÉDICTION DE LA CROISSANCE DE BACTÉRIES COLIFORMES DANS DES ENVIRONNEMENTS

(30) Priority: 26.07.2024 IN 202421056902
(43) Date of publication of application: 28.01.2026
(73) Proprietor: Tata Consultancy Services Limited, Maharashtra (IN)
(72) Inventor: BENSAM, Febin, 695581 Thiruvananthapuram (IN); SEELAN, Jim, 695581 Thiruvananthapuram (IN); MATHEW, Jos, 682042 Kochi (IN); JULIUS, Jason Lenox, 695581 Thiruvananthapuram (IN); VELLAYARAJ, Shreedhar, 600 096 Chennai (IN); VILLIAMBAKKAM CHAKRAVARTHY, Krishnan, 600 096 Chennai (IN); SAM VICTOR, Shobin Sam, 695581 Trivandrum (IN); SATHYA NARAYAN, Padmakumar, 695581 Trivandrum (IN)
(74) Representative: Goddar, Heinz J.

(56) References cited:
- WO-A1-2023/115159
- WO-A1-2024/023823
- TW-B- I 799 284
- MOHAMMED HADI ET AL: "Emulating process-based water quality modelling in water source reservoirs using machine learning", JOURNAL OF HYDROLOGY, ELSEVIER, AMSTERDAM, NL, vol. 609, 3 March 2022 (2022-03-03), XP087071036, ISSN: 0022-1694, [retrieved on 20220303], DOI: 10.1016/J.JHYDROL.2022.127675

## Description

### CROSS-REFERENCE TO RELATED APPLICATIONS AND PRIORITY

The present application claims priority to Indian application no. 202421056902, filed on July 26, 2024.

### TECHNICAL FIELD

The disclosure herein generally relates to water quality monitoring, and, more particularly, to a method and system for predicting growth of coliform bacteria in environments.

### BACKGROUND

Existing predictive models often suffer from being static in nature. These predictive models typically employ fixed algorithms and parameters, which may not sufficiently account for the dynamic nature of environmental conditions and microbial populations. Environmental conditions in water systems can vary widely over time and space, and microbial populations exhibit complex adaptive behaviors in response to these changes. Static models fail to adapt to these dynamic conditions, compromising their predictive accuracy and limiting their utility in guiding proactive management strategies.

Most of the existing approaches rely on datasets that are often fragmented and lack integration across various sources of environmental data. These datasets typically include parameters such as temperature, nutrient levels, water flow dynamics, and microbial counts. However, the failure to comprehensively integrate these diverse data sources hampers the ability to accurately predict Escherichia coli (E. coli) growth dynamics in water environments. As a result, the predictive models derived from such data may not adequately capture the complex interactions between environmental variables and microbial behavior, leading to inaccurate predictions and unreliable risk assessments.

TWI799284B discloses a related water quality prediction and sterilization control system.

### SUMMARY

Embodiments of the present disclosure present technological improvements as solutions to one or more of the above-mentioned technical problems recognized by the inventors in conventional systems. For example, in one embodiment, a method for predicting growth of coliform bacteria in environments is provided. The method includes receiving, via one or more hardware processors, (i) data from one or more sensors specific to a water sample collected from water flowing in a pipe and stored in an apparatus (ii) information specific to Geographic Information System (GIS) pertaining to a region from where the water sample is collected and (iii) data specific to one or more process parameters pertaining to a water storage and a processing unit; mapping, via the one or more hardware processors, the data received from one or more sensors with the information specific to Geographic Information System (GIS) to obtain one or more contextual information; preprocessing, via the one or more hardware processors, the obtained one or more contextual information along with the data specific to the one or more process parameters using one or more data preprocessing techniques; feeding, via the one or more hardware processors, the preprocessed data into one or more trained Machine Learning (ML) models, wherein the one or more ML models are trained using one or more ML training techniques using data from a dataset; predicting, via the one or more hardware processors, a likelihood of Escherichia coli (E. coli) using the trained one or more ML Models; obtaining, via the one or more hardware processors, a correlation matrix of one or more parameters comprised in the preprocessed data and the predicted likelihood of Escherichia coli (E. coli); and assessing, via the one or more hardware processors, contamination risks associated with the water sample based on the correlation matrix obtained.

In another aspect, there is provided a system for predicting growth of coliform bacteria in environments. The system comprises: a memory storing instructions; one or more communication interfaces; and one or more hardware processors coupled to the memory via the one or more communication interfaces, wherein the one or more hardware processors are configured by the instructions to: receive (i) data from one or more sensors specific to a water sample collected from water flowing in a pipe and stored in an apparatus (ii) information specific to Geographic Information System (GIS) pertaining to a region from where the water sample is collected and (iii) data specific to one or more process parameters pertaining to a water storage and a processing unit. The system further includes mapping the data received from one or more sensors with the information specific to Geographic Information System (GIS) to obtain one or more contextual information; preprocessing the obtained one or more contextual information along with the data specific to the one or more process parameters using one or more data preprocessing techniques; feeding the preprocessed data into one or more trained Machine Learning (ML) models, wherein the one or more ML models are trained using one or more ML training techniques using data from a dataset; predicting a likelihood of Escherichia coli (E. coli) using the trained one or more ML Models; obtaining a correlation matrix of one or more parameters comprised in the preprocessed data and the predicted likelihood of Escherichia coli (E. coli); and assessing contamination risks associated with the water sample based on the correlation matrix obtained.

In yet another aspect, there are provided one or more non-transitory machine-readable information storage mediums comprising one or more instructions which when executed by one or more hardware processors cause receiving (i) data from one or more sensors specific to a water sample collected from water flowing in a pipe and stored in an apparatus (ii) information specific to Geographic Information System (GIS) pertaining to a region from where the water sample is collected and (iii) data specific to one or more process parameters pertaining to a water storage and a processing unit; mapping the data received from one or more sensors with the information specific to Geographic Information System (GIS) to obtain one or more contextual information; preprocessing the obtained one or more contextual information along with the data specific to the one or more process parameters using one or more data preprocessing techniques; feeding the preprocessed data into one or more trained Machine Learning (ML) models, wherein the one or more ML models are trained using one or more ML training techniques using data from a dataset; predicting a likelihood of Escherichia coli (E. coli) using the trained one or more ML Models; obtaining a correlation matrix of one or more parameters comprised in the preprocessed data and the predicted likelihood of Escherichia coli (E. coli); and assessing contamination risks associated with the water sample based on the correlation matrix obtained.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary system for predicting growth of coliform bacteria in environments, according to some embodiments of the present disclosure.
FIG. 2 illustrates a functional block diagram of the system for predicting growth of coliform bacteria in environments, according to some embodiments of the present disclosure.
FIGS. 3A and 3B are flow diagrams illustrating the steps involved in the method for predicting growth of coliform bacteria in environments, according to some embodiments of the present disclosure.
FIG. 4 illustrates a correlation matrix of the one or more sensor data and an E. coli likelihood with Geographic Information System (GIS) data, in conjunction with the method for predicting growth of coliform bacteria in environments, according to some embodiments of the present disclosure.
FIG. 5 illustrates a correlation matrix of one or more sensor data and an E. coli likelihood, in conjunction with the method for predicting growth of coliform bacteria in environments, according to some embodiments of the present disclosure.
FIGS. 6A and 6B illustrates a comparison between a Linear Regression algorithm and a Random Forest algorithm in capturing the relationships between the one or more sensor data and the likelihood of E. coli growth, in conjunction with the method for predicting growth of coliform bacteria in environments, according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the claimed invention.

Existing methods for predicting growth of coliform bacteria lack consideration for spatial factors. While some approaches acknowledge the importance of geographical factors in microbial contamination, such as land use patterns and proximity to pollution sources, they often lack robust spatial analysis capabilities. Spatial heterogeneity in microbial distribution within water environments can have a significant impact on contamination risks and management strategies. However, existing methods may overlook these spatial dynamics, resulting in suboptimal predictions and management interventions.

Many current predictive models rely heavily on single machine learning algorithms or simplistic statistical methods. While these approaches may yield useful insights, they often lack the robustness and flexibility needed to handle the complexity of environmental systems and microbial dynamics. By relying solely on a single algorithm or method, these models may fail to capture the full range of interactions between environmental variables and microbial growth, limiting their predictive capability.

Existing methods often overlook critical process parameters within water treatment and distribution systems. Factors such as disinfection efficacy, hydraulic conditions, and pipe material characteristics can significantly influence microbial survival and proliferation. However, current approaches may focus primarily on environmental factors and neglect these process parameters, leading to incomplete risk assessments and suboptimal management decisions. The challenges associated with existing methods and technologies for predicting and managing E. coli growth in water environments highlights the need for innovative approaches that address the limitations of current approaches and provide actionable insights to support proactive water quality management efforts.

To overcome the challenges of the conventional approaches, embodiments herein provide a method and system for predicting growth of coliform bacteria in various environments. The present disclosure receives data from one or more sensors specific to a water sample collected from water flowing in a pipe, information specific to Geographic Information System and data specific to one or more process parameters. The data received from one or more sensors is mapped with the information specific to GIS. Mapped data along with the data specific to the one or more process parameters process using one or more data preprocessing techniques. The preprocessed data is fed into one or more trained Machine Learning (ML) models to predict likelihood of Escherichia coli (E. coli). A correlation matrix of one or more parameters comprised in the preprocessed data and the predicted likelihood of E. coli is obtained. Contamination risks associated with the water sample are assessed based on the correlation matrix obtained.

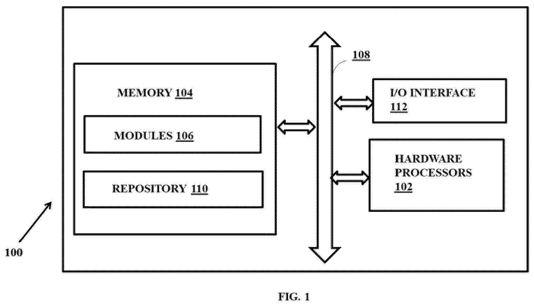

Referring now to the drawings, and more particularly to FIG. 1 through 6B, where similar reference characters denote corresponding features consistently throughout the figures, there are shown preferred embodiments, and these embodiments are described in the context of the following exemplary system and/or method.

FIG. 1 illustrates an exemplary system for predicting growth of coliform bacteria in environments, according to some embodiments of the present disclosure. In an embodiment, the system 100 includes or is otherwise in communication with hardware processors 102, at least one memory such as a memory 104, and an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable the system 100 to communicate with other devices, such as web servers, and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.

The one or more hardware processors 102 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 102 is configured to fetch and execute computer-readable instructions stored in memory 104.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 104 includes a plurality of modules 106. The memory 104 also includes a data repository (or repository) 110 for storing data processed, received, and generated by the plurality of modules 106.

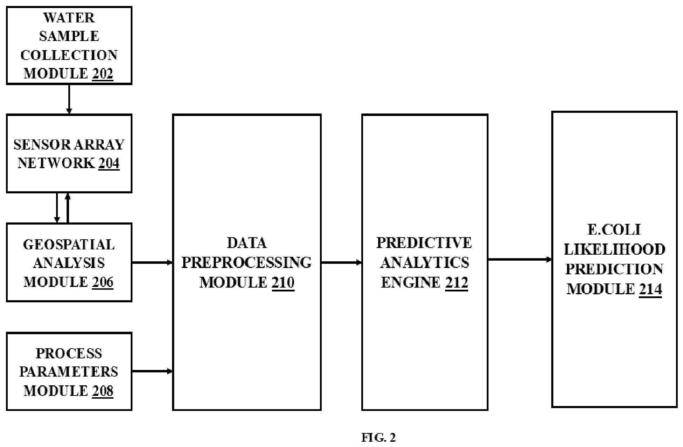

The plurality of modules 106 includes programs or coded instructions that supplement applications or functions performed by the system 100 for predicting growth of coliform bacteria in environments. The plurality of modules 106, amongst other things, can include routines, programs, objects, components, and data structures, which perform particular tasks or implement particular abstract data types. The plurality of modules 106 may also be used as signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the system 100 for predicting growth of coliform bacteria in environments. In an embodiment, the modules 106 include a water sample collection module 202, a sensor array network 204, a Geospatial Analysis module 206, a process parameters module 208, a data preprocessing module 210, a predictive analytics engine 212, and a E. coli likelihood prediction module 214. The modules are depicted in FIG. 2. These modules that are depicted in FIG. 2 are implemented as at least one of a logically self-contained part of a software program, a self-contained hardware component, and/or, a self-contained hardware component with a logically self-contained part of a software program embedded into each of the hardware component that when executed perform the above method described herein, in one embodiment of the present disclosure.

The data repository (or repository) 110 may include a plurality of abstracted pieces of code for refinement and data that is processed, received, or generated as a result of the execution of the module(s) 106.

Although the data repository 110 is shown internal to the system 100, it will be noted that, in alternate embodiments, the data repository 110 can also be implemented external to the system 100, where the data repository 110 may be stored within a database (repository 110) communicatively coupled to the system 100. The data contained within such an external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS).

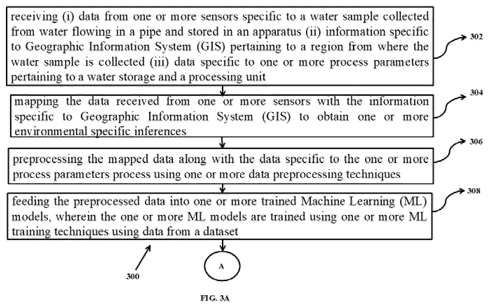

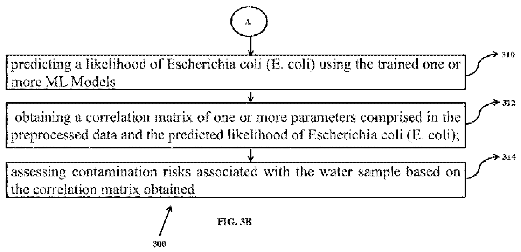

FIGS. 3A and 3B are flow diagrams illustrating a method for predicting growth of coliform bacteria in environments using the systems 100 of FIGS. 1-2, according to some embodiments of the present disclosure. Steps of the method of FIGS. 3A and 3B shall be described in conjunction with the components of FIG. 2. At step 302 of the method 300, the one or more hardware processors 102 receives i) data from one or more sensors (represented by the sensor array network 204) specific to a water sample collected from water flowing in a pipe (represented by the water sample collection module 202) and stored in an apparatus (ii) information specific to Geographic Information System (GIS) (represented by the Geospatial Analysis module 206) pertaining to a region from where the water sample is collected and (iii) data specific to one or more process parameters (represented by the process parameters module 208) pertaining to a water storage and a processing unit. The apparatus comprises ISCO 6712 portable sampler and YSI EXO2 multiparameter sonde capable of collecting the water samples. The environments can be Municipal water supply and distribution systems, Residential water systems, Industrial water systems, Agricultural water sources and the like.

The sensor array network 204 consists of an array of advanced sensors capable of collecting various environmental data. Data is collected from various types of sensors and makes inferences out of them which is used in the prediction of E. coli likelihood. The one or more sensors used to collect data are temperature sensors, humidity sensors, pH sensors, dissolved oxygen sensors, turbidity sensors, conductivity sensors, nutrient sensors, flow meters, pressure sensors, chlorine sensors, Ultraviolet (UV) sensors, chemical sensors, biofilm sensors, optical sensors and geospatial sensors. The details of the one or more sensors are described in the following sections.

Temperature Sensors: These types of sensors are deployed in water treatment plants, storage tanks, and distribution pipes to continuously monitor water temperature. Example: 30°C (30 degree Celsius). Impact: E. coli thrives in temperatures between 20°C and 45°C, with optimal growth at 37°C. A temperature of 30°C is conducive to moderate bacterial growth. In a water storage tank, this temperature triggers alerts indicating favorable conditions for E. coli proliferation.

Humidity Sensors: These types of sensors are installed in the ambient environment around water storage facilities and distribution networks to monitor humidity levels that may influence microbial growth in exposed areas. Example: 85 Relative Humidity. Impact: High humidity levels can support biofilm formation, which provides a habitat for E. coli. For instance, high humidity in underground pipes may create microenvironments where E. coli can survive and multiply.

pH Sensors: These types of sensors are placed at various points in water systems, such as inflows, treatment stages, and outflows, to monitor acidity/alkalinity. Example: pH 6.5. Impact: E. coli grows best in slightly acidic to neutral pH (6.5 to 7.5). In a corrosive pipe, a pH of 6.5 could signal optimal conditions for bacterial growth, necessitating close monitoring to prevent contamination.

Dissolved Oxygen Sensors: These types of sensors are used to measure oxygen levels in water storage tanks and natural water bodies. Example: 2 mg/L. Impact: E. coli can grow in both aerobic and anaerobic conditions. Low dissolved oxygen levels (2 mg/L) in a stagnant water tank can create anaerobic conditions, which are favorable for E. coli growth.

Turbidity Sensors: These types of sensors are deployed at critical points in water systems to detect particulate matter. Example: 12 NTU (Nephelometric Turbidity Units). Impact: High turbidity indicates suspended particles that can protect E. coli from disinfection processes. Increased turbidity in a distribution pipe may signal the presence of sediment harboring E. coli, indicating potential contamination.

Conductivity Sensors: These types of sensors are used for monitoring ion concentration, providing insights into water quality and potential contamination. Example: 700. S/cm (microsiemens per centimeter). Impact: High conductivity suggests the presence of dissolved ions and nutrients. In pipes, a conductivity of 700. S/cm might indicate corrosive salts, which facilitate E. coli growth by providing a supportive chemical environment.

Nutrient Sensors: These types of sensors are used to detect key nutrients (e.g., nitrogen, phosphorus) in water systems. Example: 4mg/L Nitrogen. Impact: Elevated nutrient levels, particularly nitrogen, promote E. coli growth. Agricultural runoff entering a water reservoir with 4 mg/L nitrogen can create nutrient-rich conditions conducive to bacterial proliferation.

Flow Meters: These types of meters are used to measure water flow rates in treatment plants and distribution networks. Example: 0.05 m/s (meters per second). Impact: Low flow rates can lead to stagnation, which favors E. coli accumulation. Monitoring flow dynamics in pipes helps identify such low-flow areas, prompting targeted maintenance to prevent bacterial buildup.

Pressure Sensors: These types of sensors are installed in distribution systems to detect pressure drops indicative of leaks. Example: 1.5 bar. Impact: A sudden drop in pressure can indicate leaks in the distribution system, potentially introducing contaminants. Detecting a pressure drop to 1.5 bar from a normal operating pressure of 2.5 bar can alert to possible points of entry for E. coli.

Chlorine Sensors: These types of sensors are used to monitor residual chlorine levels to ensure effective disinfection. Example: 0.1 mg/L Residual Chlorine. Impact: Effective disinfection requires sufficient chlorine levels. Decreasing residual chlorine to 0.1 mg/L in a distribution pipe may allow E. coli to persist, indicating the need for increased disinfection measures.

Ultraviolet (UV) Sensors: These types of sensors are used to measure Ultraviolet (UV) intensity in disinfection systems to ensure microbial inactivation. Example: 30 mJ/cm² UV Dose. Impact: Ultraviolet (UV) sensors measure the intensity of UV light in disinfection systems. An Ultraviolet (UV) dose of 30 mJ/cm² ensures the microbial inactivation of E. coli, providing an additional layer of protection in water treatment plants.

Chemical Sensors: These types of sensors are used to detect specific contaminants, such as heavy metals and pesticides. Example: 0.05 mg/L Pesticides. Impact: High levels of pesticides (0.05 mg/L) in agricultural runoff can compromise water quality and facilitate E. coli growth by altering the chemical environment and reducing the efficacy of natural microbial controls.

Biofilm Sensors: These types of sensors are used to detect biofilm formation in pipes and tanks. Example: Biofilm Thickness 50.m. Impact: Biofilms provide a protected environment for E. coli. Detecting biofilm thickness of 50 .m in pipes indicates areas where E. coli may thrive, necessitating targeted cleaning and disinfection.

Optical Sensors: These types of sensors use fluorescence and absorbance to detect microbial presence. Example: Fluorescence Intensity 300 RFU (Relative Fluorescence Units). Impact: Optical sensors detect microbial presence via fluorescence. High fluorescence intensity (300 RFU) in real-time monitoring stations can provide immediate alerts for E. coli contamination, enabling rapid response.

Geospatial Sensors: These types of sensors use GPS and GIS to track sensor locations and contamination sources. Example: GPS Coordinates (37.7749. N, 122.4194. W). Impact: Geospatial sensors track sensor locations and contamination sources. Mapping contamination hotspots in a city's water distribution network helps target interventions precisely, reducing the spread of E. coli.

Geospatial Analysis module 206: The Geospatial Analysis module 206 processes spatial data related to terrain characteristics, land use patterns, proximity to contamination sources, and hydrological features. The Geospatial Analysis module 206 module employs Geographic Information System (GIS) technology to collect spatial data and identify spatial patterns relevant to coliform growth as well as terrain and environmental characteristics. They mostly rely on satellite information to understand about the environment and the surrounding characteristics.

Geospatial Information Systems (GIS) for Contextual Analysis: GIS is integrated into the system 100 to provide a spatial context to the sensor data, enhancing the predictive analytics of E. coli growth by considering geographic and environmental factors.

Spatial Data Collection: Geospatial Sensors: Devices like GPS and GIS-enabled sensors track the exact locations of water quality measurements and contamination sources.

Environmental Data: Data on land use, soil type, topography, and hydrology are collected, which can influence water quality and E. coli presence.

GIS Mapping and Analysis: Spatial Correlation: GIS helps in correlating sensor data with geographic factors. For instance, areas near agricultural fields may show higher nutrient levels due to runoff.

Hotspot Identification: Spatial analysis tools identify hotspots where E. coli contamination is more likely. This includes mapping areas with frequent pipe leaks or stagnant water zones.

Temporal Analysis: Spatial data is combined with temporal data to understand how E. coli contamination evolves over time in different locations. Similar to testing out the data from various sensors and coming out with inference among the relationship between different parameters and the corresponding likelihood of E. coli likelihood.

Process parameters module 208: The process parameters module 208 includes algorithms and data to integrate process parameters relevant to water treatment and distribution systems. These process parameters include data such as flow rates, residence times, disinfection efficacy, and pipe material characteristics. Incorporating process parameter data enhances the understanding of contamination risks and supports proactive management strategies.

At step 304 of the method 300, the one or more hardware processors 102 map the data received from one or more sensors with the information specific to Geographic Information System (GIS) to obtain one or more contextual information. The one or more contextual information refers to proximity to contamination sources, land use patterns (agricultural, industrial, residential, urban), topographical features (elevation, slope,), climate and weather patterns, soil types and the like. To enhance the accuracy of the predictions of the likelihood of E. coli, the system 100 incorporates geospatial analysis using GIS technology. By mapping one or more sensors data to specific geographical locations, how local environmental conditions, such as rainfall or proximity to potential contamination sources, influence E. coli growth can be assessed. This contextual information is crucial for understanding the spatial dynamics of bacterial proliferation.

At step 306 of the method 300, the data preprocessing module 210 executed via the one or more hardware processors 102 pre-processes the obtained one or more contextual information along with the data specific to the one or more process parameters process using one or more data preprocessing techniques. The one or more data preprocessing techniques include data cleaning (handling missing values), data normalization and standardization, data transformation (feature engineering, temporal aggregation), integration. The data from the sensor array network 204, the Geospatial Analysis module 206 and the process parameters module 208 are integrated into a centralized data repository. This integration ensures that data from diverse sources are normalized, processed and made available for analysis.

Data preprocessing is a critical step in the implementation of the predictive analytics system for monitoring E. coli growth. Data preprocessing phase involves several processes to clean, transform, and organize the collected raw data from various sensors, ensuring it is suitable for analysis and modeling.
1. Data Cleaning: Error detection and correction: Raw sensor data often contains errors due to malfunctions, environmental interference, or transmission issues. Techniques such as outlier detection (known in the art), anomaly detection (known in the art), and rule-based validation (known in the art) are applied to identify and correct these errors. For example, an abrupt spike in temperature readings that deviates significantly from the norm might indicate a sensor malfunction and can be flagged for further inspection. Missing Data Handling: Sensors may occasionally fail to transmit data, resulting in gaps. Missing values can be addressed using interpolation methods, such as linear interpolation for continuous data, or by using statistical techniques (known in the art) like mean, median, or mode imputation. For more complex scenarios, machine learning-based imputation methods can be employed.
2. Data normalization: Data normalization ensures that data from different sensors are on a similar scale, which is crucial for algorithms that are sensitive to the magnitude of data. For instance, temperature readings in Celsius and dissolved oxygen levels in milligrams per liter are normalized to a common range, typically between 0 and 1, using min-max normalization.
   Data standardization: Data standardization includes conversion of data to have a mean of zero and a standard deviation of one. This is particularly useful for algorithms that assume normally distributed data. The data standardization helps in reducing biases due to different scales and units of measurements.
3. Data transformation includes performing feature engineering, which involves creating new features from raw data that can enhance the predictive power of the machine learning models. For example, combining temperature and humidity data to create a new feature that represents the heat index, which can provide better insights into environmental conditions conducive to E. coli growth.
   Temporal Aggregation: Sensor data is often collected at high frequencies. Aggregating this data into meaningful time intervals (e.g., hourly, daily) helps in reducing noise and focusing on significant trends. This is done through statistical aggregation functions such as mean, median, or summing over specified periods.
4. Data integration: Data from multiple sources (e.g., different types of sensors, external environmental data) are merged to create a comprehensive dataset. This involves aligning data on common time stamps or geographic coordinates, ensuring that all relevant variables are included in the analysis.
   Handling Geospatial Data: Integrating GPS coordinates with sensor data to provide spatial context. Geographic Information System (GIS) tools are used to map sensor locations and correlate spatial data with environmental parameters and E. coli growth patterns.
5. Data reduction: Techniques such as Principal Component Analysis (PCA) (known in the art) or t-Distributed Stochastic Neighbor Embedding (t-SNE) (known in the art) are used to reduce the number of features while retaining the most important information. This helps in speeding up the computation and improving model performance by eliminating redundant or less significant variables. Sampling: In cases where the dataset is very large, sampling methods like stratified sampling or random sampling are used to create a manageable subset of data for initial analysis and model training.

Hence, data from the one or more sensors is collected in real-time and transmitted to a processing unit. The processing unit can be edge devices, cloud based platforms, or on premises servers. Here, from the one or more sensors data undergoes initial aggregation, where readings from different sensors at various locations are compiled into a cohesive dataset. This aggregated data provides a comprehensive view of the water system's current state, capturing both environmental conditions and process parameters.

At step 308 of the method 300, the predictive analytics engine 212 executed via the one or more hardware processors 102 feeds the preprocessed data into one or more trained Machine Learning (ML) models. The one or more ML models are trained using one or more ML training techniques. The predictive analytics engine 212 has one or more machine learning algorithms to analyze integrated data and generate predictions for identifying anomalies.

The training of the one or more ML models comprises the following steps. Splitting the data taken from a combination of dataset acquired by the sensors as well as other opensource data available into a training data and a testing data. The data taken from the dataset comprise (i) various types of information obtained from sensors such as temperature sensors, humidity sensors, pH sensors, dissolved Oxygen sensors, nutrient level, flow rate, pressure, turbidity, color and the like (ii) GIS data such as Altitude, Distance to contamination source, land use types (agricultural, industrial, residential, urban) and (iii) process parameter such as data specific to water storage conditions and specific to processing unit conditions(treatment processes, maintenance schedules). The one or more ML models are trained using the training data. Hyperparameter tuning is performed on the trained one or more Machine Learning models to obtain one or more values for one or more parameters comprised in the trained one or more Machine Learning models. The one or more values corresponding to the one or more parameters comprised in the trained one or more Machine Learning models are optimized. The trained one or more Machine Learning models are evaluated by calculating one or more performance metrics. The trained one or more Machine Learning models are compared based on the calculated one or more performance metrics.

Feature Extraction: From the aggregated dataset comprising the data from the one or more sensors, GIS data, data specific to the one or more process parameters, relevant features are extracted that are known to influence E. coli growth. These features include temperature fluctuations, humidity levels, pH variations, dissolved oxygen levels, conductivity readings, nutrient concentrations, water flow rates, pressure changes, turbidity levels, chlorine residuals, and the like. These features further include GIS data such as altitude, distance to contamination source, land use types, other process parameter features such as treatment processes, maintenance schedules.

Machine Learning Models: The preprocessed data feeds into various machine learning models to predict E. coli growth and contamination risks. These models learn patterns and relationships between environmental and process parameters (including GIS data as well as the other sensor data such Total Dissolved Solids (TDS), pH, etc.) and E. coli presence.

Regression Models (known in the art): Linear Regression: Models the relationship between dependent and independent variables linearly, predicting E. coli concentration based on factors like temperature and pH.
Polynomial Regression (known in the art): Captures non-linear relationships by including polynomial terms of predictor variables, useful for modeling complex interactions between environmental factors and bacterial growth.
Classification Models (known in the art): Logistic Regression: Used for binary classification tasks, such as predicting whether E. coli levels will exceed safety thresholds based on current conditions.

Decision Trees and Random Forests (known in the art): Use decision rules to classify data. Random forests combine multiple decision trees to enhance accuracy and robustness, predicting contamination events based on diverse environmental inputs.

Support Vector Machines (SVM) (known in the art): Finds the optimal hyperplane that separates classes, effective for predicting contamination risks based on multiple features.
Clustering Algorithms (known in the art): K-Means Clustering: Groups data based on feature similarity, identifying natural clusters of contamination patterns.
Hierarchical Clustering (known in the art): Builds a hierarchy of clusters to explore nested relationships in the data. that allows for the identification of both the larger groups and the finer sub-groups within the data using a tree-like structure (dendrogram) that can provide insights into the different levels of contamination pattern.

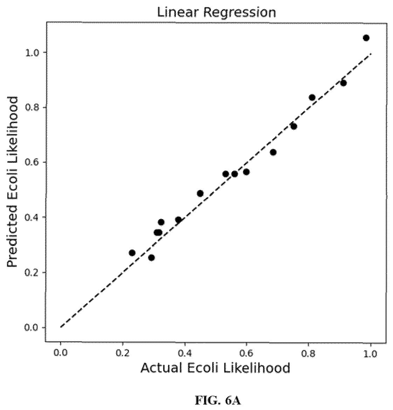

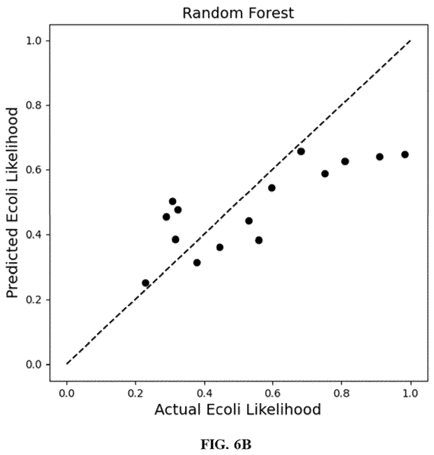

The one or more ML models trained in the present disclosure are evaluated with test data identified from the individual sensors to understand the performance of each of the one or more ML models (specifically Linear regression model and Decision Tree model). The one or more ML Models that are created with Linear regression or any other statistical models can be used. They can be a simple Artificial Neural Network (ANN) which is a deep learning algorithm. Fig. 6A and Fig. 6B created was not with an ANN but with a simple linear statistical model. Further, while implementing in real-world scenario a neural network is helpful in improving the accuracy and efficiency. The test data has been taken across sensors such as Temperature, Humidity, pH, Dissolved Oxygen, Conductivity, Nutrient Level, Flow Rate, Pressure, Turbidity, Chlorine. The data specific to the one or more sensors, information specific to the GIS and the data specific to the one or more parameters is collected and preprocessed and fed into the algorithm to predict the likelihood of E. coli.

At step 310 of the method 300, the E. coli likelihood prediction module 214 executed via the one or more hardware processors 102 predicts a likelihood of Escherichia coli (E. coli) using the trained one or more ML Models.

At step 312 of the method 300, the one or more hardware processors 102 obtains a correlation matrix of one or more parameters comprised in the preprocessed data and the predicted likelihood of Escherichia coli (E. coli). The one or more parameters comprised in the preprocessed data include data specific to the one or more sensors, the information specific to the GIS and the data specific to the one or more process parameters.

At step 314 of the method 300, the one or more hardware processors 102 assesses contamination risks associated with the water sample based on the correlation matrix obtained.

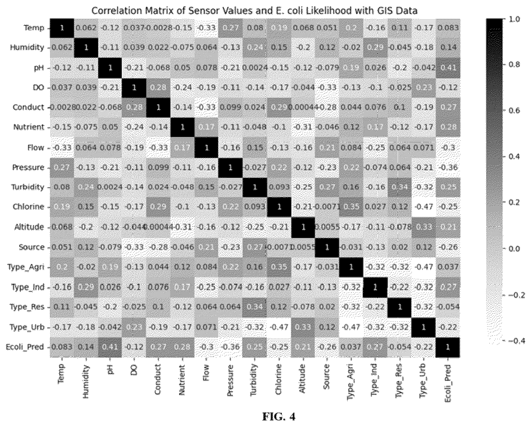

FIG. 4 illustrates a correlation matrix of the one or more sensor data and an E. coli likelihood with Geographic Information System GIS data, in conjunction with the method for predicting growth of coliform bacteria in environments, according to some embodiments of the present disclosure.

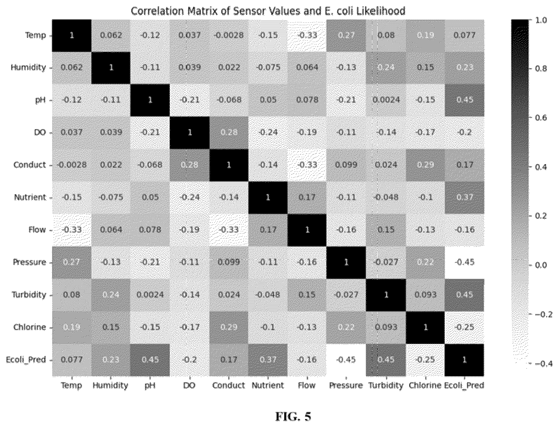

FIG. 5 illustrates a correlation matrix of one or more sensor data and an E. coli likelihood, in conjunction with the method for predicting growth of coliform bacteria in environments, according to some embodiments of the present disclosure.

Correlation matrix of the one or more sensor data and the E. coli Likelihood: Correlation Coefficient (r): This value ranges from -1 to 1, indicating the strength and direction of a linear relationship between two variables.
1: Perfect positive correlation. As one variable increases, the other variable increases.
-1: Perfect negative correlation. As one variable increases, the other variable decreases. 0: No linear correlation between the variables.

The correlation matrix displays the correlation coefficients between each pair of variables (the one or more parameters comprised in the preprocessed data). The color scale on the right indicates the strength of the correlation, with black color representing high positive correlation, white color representing high negative correlation, and shades in between representing weaker correlations.

### Example inferences that can be made are,

1. Temperature: - Humidity: Weak positive correlation (0.062).
   - pH: Weak negative correlation (-0.12).
   - Dissolved Oxygen: Very weak positive correlation (0.037).
   - E. coli Likelihood: Weak positive correlation (0.077).
   Temperature has a weak positive impact on E. coli growth. As temperature increases, E. coli growth likelihood slightly increases, which is consistent with known bacterial behavior in warmer environments.
2. Humidity
   - Turbidity: Weak positive correlation (0.24).
   - Chlorine: Weak positive correlation (0.15).
   - E. coli Likelihood: Moderate positive correlation (0.23).
   Higher humidity levels can create environments that are more conducive to bacterial growth, indicated by a moderate positive correlation with E. coli likelihood.

FIGS. 6A and 6B illustrates a comparison between a Linear Regression algorithm and a Random Forest algorithm in capturing the relationships between the one or more sensor data and the likelihood of E. coli growth, in conjunction with the method for predicting growth of coliform bacteria in environments, according to some embodiments of the present disclosure.

Comparison between the Linear Regression algorithm and Random Forest algorithm:
Linear Regression: The predicted values are closely aligned with the actual values, indicating that the Linear Regression model is performing well. The small spread around the black dashed line suggests low error and high predictive accuracy.
Random Forest: The predicted values show greater deviation from the actual values, with a larger spread around the black dashed line. This suggests that the Random Forest model has higher error and lower predictive accuracy compared to the Linear Regression model. Therefore, it can be inferred that Linear Regression is the better algorithm in this case, as evidenced by the closer alignment of the predicted values with the actual values and the smaller residual errors compared to the Random Forest model. This suggests Linear Regression is more effective in capturing the relationships between the sensor data and the likelihood of E. coli growth in this dataset.

Land Use Types and E. coli Likelihood:
An agricultural land (0.27) that has a positive correlation indicates agricultural areas are likely associated with higher E. coli presence. An industrial land (0.27) that has positive correlation, shows industrial areas also have a significant impact. A residential (0.054) land that has slight positive correlation indicates less significant than other land use types. An urban (-0.22) land that has negative correlation indicates urban areas might have better management against E. coli contamination.

Interpretation of correlations among sensors and GIS data: Temperature and Conductivity (0.27): Moderate positive correlation, suggesting that temperature changes might affect water conductivity.

Flow Rate and Nutrient Level (0.17): Positive correlation indicates areas with higher flow rates might also have higher nutrient levels.
Turbidity and Conductivity (0.24): Positive correlation, as more turbid water is likely to have higher conductivity.
Pressure and Altitude (0.33): Positive correlation, likely because higher altitudes often have higher pressure measurements.
Chlorine and Urban Land Use (-0.47): Strong negative correlation, indicating urban areas may use more chlorine for disinfection.
Industrial Land Use and pH (0.29): Positive correlation, suggesting industrial areas might influence water pH levels.

The correlation matrix shows that multiple factors influence the likelihood of E. coli presence in water. Sensor values (the one or more sensor data) such as pH, nutrient levels, and conductivity, along with GIS factors like land use type and proximity to contamination sources, play significant roles. By understanding these relationships, effective monitoring and management strategies can be developed to mitigate E. coli contamination in environments.

The method and system of the present disclosure uniquely combine environmental sensor data with Geographic Information System (GIS) parameters to predict the likelihood of E. coli contamination. The method of the present disclosure integrates sensor readings, such as temperature, humidity, and pH, with geographical and anthropogenic factors like altitude, distance to contamination sources, and land use types. By contextualizing environmental data within its geographical settings, the present disclosure provides a comprehensive understanding of the factors influencing E. coli's presence. In the present disclosure, advanced data processing techniques including dynamic normalization and feature engineering are employed (e.g., techniques are stored in the system 100 and invoked for execution of the method of the present disclosure) to convert raw data into meaningful inputs for the one or more ML models. Specifically, the Artificial Neural Network models on top of the statistical techniques such as linear regression.

Use case example: Monitoring a city's water distribution network. Data: GIS data includes sensor locations, land use patterns, rainfall data, and historical contamination reports. Inference: GIS mapping identifies a correlation between recent heavy rainfall and increased nutrient levels in runoff areas. The predictive model highlights potential contamination hotspots, allowing for targeted interventions.

There are several applications which can be implemented to analyze the growth and proliferation of E. coli.
1. Analyzing corrosive nature of pipes: By deploying conductivity and chemical sensors within pipes, the system 100 detects increased levels of corrosive substances like salts and heavy metals. This data is combined with flow meters and pressure sensors, to identify areas where pipe corrosion is likely, creating conditions favorable for E. coli growth due to pipe material degradation. For example, Temperature: 32.C; pH Level: 6.8; Dissolved Oxygen: 3 mg/L; Conductivity: 600. S/cm; Water Flow Rate: 0.05 m/s. Impact: The moderately high temperature (32.C) and pH (6.8) are within the optimal range for E. coli growth. Low dissolved oxygen (3 mg/L) supports both aerobic and anaerobic growth phases. High conductivity (600. S/cm) indicates the presence of nutrients and ions that can further support growth. The very low water flow rate (0.05 m/s) creates stagnation in areas, which can lead to localized hotspots of E. coli proliferation.
2. Monitoring water stored in tanks: Temperature and dissolved oxygen sensors in water storage tanks provide critical data on conditions that could promote bacterial growth. For instance, a rise in water temperature and a drop in dissolved oxygen levels could trigger alerts for potential E. coli proliferation, prompting preemptive disinfection measures. Example: Temperature: 28.C; pH Level: 7.2; Dissolved Oxygen: 5 mg/L; Nutrient Level (Nitrogen): 2.5 mg/L; Turbidity: 8 NTU. Impact: The temperature (28.C) is suitable for moderate growth, slightly below the optimal but still conducive. A neutral pH (7.2) is ideal for E. coli. Sufficient dissolved oxygen (5 mg/L) supports aerobic metabolism. High nutrient levels (2.5 mg/L nitrogen) provide ample resources for bacterial growth. Increased turbidity (8 NTU) suggests the presence of particulate matter that can shield E. coli from disinfectants, promoting survival.
3. Assessing outside environmental variables: Environmental sensors for temperature, humidity, and rainfall data provide insights into how external conditions impact water quality. For example, high rainfall leads to runoff entering water sources, carrying nutrients and contaminants that promote E. coli growth, which can be predicted using nutrient and turbidity sensors. For example: External Temperature: 24.C; Humidity: 80; Rainfall: 50 mm (about 1.97 in) in the last 24 hours; Nutrient Level (Nitrogen): 3 mg/L; Turbidity: 10 Nephelometric Turbidity unit (NTU) Impact: The external temperature (24.C) is slightly below the optimal range for E. coli growth but can still support moderate bacterial activity. High humidity (80) can contribute to the formation of biofilms in exposed areas, providing a habitat for E. coli. Significant rainfall (50 mm) increases the likelihood of nutrient and contaminant runoff entering water sources. Elevated nutrient levels (3 mg/L nitrogen) from runoff provide ample food for E. coli, promoting rapid growth. High turbidity (10 NTU) indicates the presence of particulate matter, which can shield E. coli from disinfectants and enhance survival rates.
4. Incorporating process parameters in water flow: Flow meters and pressure sensors in water distribution networks help monitor hydraulic conditions. Data from these sensors can identify low-flow or stagnant areas where E. coli might accumulate, enabling targeted interventions such as increased flushing or localized disinfection. For example, Water Flow Rate: 0.03 m/s; Pressure: 1.5 bar; Temperature: 22.C; Chlorine Level: 0.1 mg/L; pH Level: 7.0 Impact: A very low flow rate (0.03 m/s) creates stagnant zones, which can lead to the accumulation of E. coli. Stagnant water is more likely to become contaminated and harder to disinfect thoroughly. A relatively low pressure (1.5 bar) might indicate potential leaks or blockages that can introduce contaminants or cause water to stagnate, further promoting E. coli growth. The moderate temperature (22.C) supports moderate bacterial growth, as it is within the tolerable range for E. coli. Low chlorine levels (0.1 mg/L) suggest insufficient disinfection, allowing E. coli to survive and potentially proliferate. A neutral pH (7.0) is ideal for E. coli growth, ensuring that the bacteria can thrive if other conditions are favorable.

The written description describes the subject matter herein to enable any person skilled in the art to make and use the embodiments. The scope of the invention is defined by the claims and may include other modifications that occur to those skilled in the art. Such other modifications are intended to be within the scope of the claimed invention if they have similar elements that do not differ from the literal language of the claims or if they include equivalent elements with insubstantial differences from the literal language of the claims.

Existing predictive models employ fixed algorithms and parameters, which may not sufficiently account for the dynamic nature of environmental conditions and microbial populations. The present disclosure integrates the sensor data into the predictive analytics system to provide real-time insights into environmental conditions and facilitate accurate forecasting of the coliform growth dynamics. Further, the present disclosure utilizes geographic information system (GIS) technology to visualize spatial data and identify spatial patterns or correlations relevant to coliform growth in water environments. Furthermore, the present disclosure implements model fusion techniques including bagging technique (known in the art) and boosting technique (known in the art) to integrate predictions from multiple algorithms and generate comprehensive forecasts, thereby enhancing the reliability of the predictive analytics system. The present disclosure incorporates process parameter data into the proposed predictive model to provide a holistic understanding of contamination risks and identify critical control points for preventive measures and corrective actions.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the claimed invention. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of the claimed invention being indicated by the following claims.

## Claims

1. A processor implemented method (300), comprising:
receiving (302), via one or more hardware processors, (i) data from one or more sensors specific to a water sample collected from water flowing in a pipe and stored in an apparatus (ii) information specific to Geographic Information System (GIS) pertaining to a region from where the water sample is collected and (iii) data specific to one or more process parameters pertaining to a water storage and a processing unit;
mapping (304), via the one or more hardware processors, the data received from one or more sensors with the information specific to Geographic Information System (GIS) to obtain one or more contextual information;
preprocessing (306), via the one or more hardware processors, the obtained one or more contextual information along with the data specific to the one or more process parameters using one or more data preprocessing techniques;
feeding (308), via the one or more hardware processors, the preprocessed data into one or more trained Machine Learning (ML) models, wherein the one or more ML models are trained using one or more ML training techniques using data from a dataset;
predicting (310), via the one or more hardware processors, a likelihood of Escherichia coli (E. coli) using the trained one or more ML Models;
obtaining (312), via the one or more hardware processors, a correlation matrix of one or more parameters comprised in the preprocessed data and the predicted likelihood of Escherichia coli (E. coli); and
assessing (314), via the one or more hardware processors, contamination risks associated with the water sample based on the correlation matrix obtained.

2. The processor implemented method as claimed in claim 1, wherein the one or more sensors comprise one or more temperature sensors, one or more humidity sensors, one or more pH sensors, one or more dissolved oxygen sensors, one or more turbidity sensors, one or more conductivity sensors, one or more nutrient sensors, one or more flow meters, one or more pressure sensors, one or more chlorine sensors, one or more UV sensors, one or more chemical sensors, one or more biofilm sensors, one or more optical sensors and one or more geospatial sensors.

3. The processor implemented method as claimed in claim 1, wherein the data specific to the GIS comprise an altitude, one or more land use types and one or more water source proximities.

4. The processor implemented method as claimed in claim 1, wherein the data specific to one or more process parameters comprise one or more flow rates, one or more retention time, a disinfection efficacy, and one or more characteristics specific to pipe material carrying water.

5. A system (100), comprising:
a memory (104) storing instructions;
one or more communication interfaces (112); and
one or more hardware processors (102) coupled to the memory (104) via the one or more communication interfaces (112), wherein the one or more hardware processors (102) are configured by the instructions to:
receive (i) data from one or more sensors specific to a water sample collected from water flowing in a pipe and stored in an apparatus (ii) information specific to Geographic Information System (GIS) pertaining to a region from where the water sample is collected and (iii) data specific to one or more process parameters pertaining to a water storage and a processing unit;
map the data received from one or more sensors with the information specific to Geographic Information System (GIS) to obtain one or more contextual information;
preprocess the obtained one or more contextual information along with the data specific to the one or more process parameters using one or more data preprocessing techniques;
feed the preprocessed data into one or more trained Machine Learning (ML) models, wherein the one or more ML models are trained using one or more ML training techniques using data from a dataset;
predict a likelihood of Escherichia coli (E. coli) using the trained one or more ML Models;
obtain a correlation matrix of one or more parameters comprised in the preprocessed data and the predicted likelihood of Escherichia coli (E. coli); and
assess contamination risks associated with the water sample based on the correlation matrix obtained.

6. The system as claimed in claim 5, wherein the one or more sensors comprise one or more temperature sensors, one or more humidity sensors, one or more pH sensors, one or more dissolved oxygen sensors, one or more turbidity sensors, one or more conductivity sensors, one or more nutrient sensors, one or more flow meters, one or more pressure sensors, one or more chlorine sensors, one or more UV sensors, one or more chemical sensors, one or more biofilm sensors, one or more optical sensors and one or more geospatial sensors.

7. The system as claimed in claim 5, wherein the data specific to Geographic Information System (GIS) comprise an altitude, one or more land use types and one or more water source proximities.

8. The system as claimed in claim 5, wherein the data specific to one or more process parameters comprise one or more flow rates, one or more retention time, a disinfection efficacy, and one or more characteristics specific to pipe material carrying water.

9. One or more non-transitory machine-readable information storage mediums comprising one or more instructions which when executed by one or more hardware processors cause:
receiving (i) data from one or more sensors specific to a water sample collected from water flowing in a pipe and stored in an apparatus (ii) information specific to Geographic Information System (GIS) pertaining to a region from where the water sample is collected and (iii) data specific to one or more process parameters pertaining to a water storage and a processing unit;
mapping the data received from one or more sensors with the information specific to Geographic Information System (GIS) to obtain one or more contextual information;
preprocessing the obtained one or more contextual information along with the data specific to the one or more process parameters using one or more data preprocessing techniques;
feeding the preprocessed data into one or more trained Machine Learning (ML) models, wherein the one or more ML models are trained using one or more ML training techniques using data from a dataset;
predicting a likelihood of Escherichia coli (E. coli) using the trained one or more ML Models;
obtaining a correlation matrix of one or more parameters comprised in the preprocessed data and the predicted likelihood of Escherichia coli (E. coli); and
assessing contamination risks associated with the water sample based on the correlation matrix obtained.

10. The one or more non-transitory machine-readable information storage mediums as claimed in claim 9, wherein the one or more sensors comprise one or more temperature sensors, one or more humidity sensors, one or more pH sensors, one or more dissolved oxygen sensors, one or more turbidity sensors, one or more conductivity sensors, one or more nutrient sensors, one or more flow meters, one or more pressure sensors, one or more chlorine sensors, one or more UV sensors, one or more chemical sensors, one or more biofilm sensors, one or more optical sensors and one or more geospatial sensors.

11. The one or more non-transitory machine-readable information storage mediums as claimed in claim 9, wherein the data specific to the GIS comprise an altitude, one or more land use types and one or more water source proximities.

12. The one or more non-transitory machine-readable information storage mediums as claimed in claim 9, wherein the data specific to one or more process parameters comprise one or more flow rates, one or more retention time, a disinfection efficacy, and one or more characteristics specific to pipe material carrying water.

## Patentansprüche

1. Prozessorimplementiertes Verfahren (300), umfassend:
Empfangen (302), über einen oder mehrere Hardwareprozessoren, (i) von Daten von einem oder mehreren Sensoren, die für eine Wasserprobe spezifisch sind, die aus Wasser gesammelt wurde, das in einem Rohr fließt und in einer Vorrichtung gespeichert wurde, (ii) von Informationen, die für ein geografisches Informationssystem (GIS) spezifisch sind, das sich auf eine Region bezieht, von der die Wasserprobe gesammelt wurde, und (iii) von Daten, die für einen oder mehrere Prozessparameter spezifisch sind, die sich auf einen Wasserspeicher und eine Verarbeitungseinheit beziehen;
Abbilden (304), über den einen oder die mehreren Hardwareprozessoren, der Daten, die von einem oder mehreren Sensoren empfangen wurden, mit den Informationen, die für das geografische Informationssystem (GIS) spezifisch sind, um eine oder mehrere Kontextinformationen zu erhalten;
Vorverarbeiten (306), über den einen oder die mehreren Hardwareprozessoren, der erhaltenen einen oder mehreren Kontextinformationen zusammen mit den Daten, die für den einen oder die mehreren Prozessparameter spezifisch sind, unter Verwendung einer oder mehrerer Datenvorverarbeitungstechniken;
Einspeisen (308), über den einen oder die mehreren Hardwareprozessoren, der vorverarbeiteten Daten in ein oder mehrere trainierte Maschinenlernmodelle (ML-Modelle), wobei das eine oder die mehreren ML-Modelle unter Verwendung einer oder mehrerer ML-Trainingstechniken unter Verwendung von Daten aus einem Datensatz trainiert werden;
Vorhersagen (310), über den einen oder die mehreren Hardwareprozessoren, einer Wahrscheinlichkeit von Escherichia coli (E. coli) unter Verwendung des trainierten einen oder der trainierten mehreren ML-Modelle;
Erhalten (312), über den einen oder die mehreren Hardwareprozessoren, einer Korrelationsmatrix von einem oder mehreren Parametern, die in den vorverarbeiteten Daten enthalten sind, und der vorhergesagten Wahrscheinlichkeit von Escherichia coli (E. coli); und
Beurteilen (314), über den einen oder die mehreren Hardwareprozessoren, von Kontaminationsrisiken, die mit der Wasserprobe assoziiert sind, basierend auf der erhaltenen Korrelationsmatrix.

2. Prozessorimplementiertes Verfahren nach Anspruch 1, wobei der eine oder die mehreren Sensoren einen oder mehrere Temperatursensoren, einen oder mehrere Feuchtigkeitssensoren, einen oder mehrere pH-Sensoren, einen oder mehrere Sensoren für gelösten Sauerstoff, einen oder mehrere Trübungssensoren, einen oder mehrere Leitfähigkeitssensoren, einen oder mehrere Nährstoffsensoren, einen oder mehrere Durchflussmesser, einen oder mehrere Drucksensoren, einen oder mehrere Chlorsensoren, einen oder mehrere UV-Sensoren, einen oder mehrere chemische Sensoren, einen oder mehrere Biofilmsensoren, einen oder mehrere optische Sensoren und einen oder mehrere georäumliche Sensoren umfassen.

3. Prozessorimplementiertes Verfahren nach Anspruch 1, wobei die Daten, die für das GIS spezifisch sind, eine Höhe, einen oder mehrere Landnutzungstypen und eine oder mehrere Wasserquellennähen umfassen.

4. Prozessorimplementiertes Verfahren nach Anspruch 1, wobei die Daten, die für einen oder mehrere Prozessparameter spezifisch sind, eine oder mehrere Durchflussraten, eine oder mehrere Retentionszeiten, eine Desinfektionswirksamkeit und eine oder mehrere Eigenschaften, die für Rohrmaterial, das Wasser trägt, spezifisch sind, umfassen.

5. System (100), umfassend:
einen Speicher (104), der Anweisungen speichert;
eine oder mehrere Kommunikationsschnittstellen (112); und
einen oder mehrere Hardwareprozessoren (102), die über die eine oder die mehreren Kommunikationsschnittstellen (112) mit dem Speicher (104) gekoppelt sind, wobei der eine oder die mehreren Hardwareprozessoren (102) durch die Anweisungen konfiguriert sind zum:
Empfangen (i) von Daten von einem oder mehreren Sensoren, die für eine Wasserprobe spezifisch sind, die aus Wasser gesammelt wurde, das in einem Rohr fließt und in einer Vorrichtung gespeichert wurde, (ii) von Informationen, die für ein geografisches Informationssystem (GIS) spezifisch sind, das sich auf eine Region bezieht, von der die Wasserprobe gesammelt wurde, und (iii) von Daten, die für einen oder mehrere Prozessparameter spezifisch sind, die sich auf einen Wasserspeicher und eine Verarbeitungseinheit beziehen;
Abbilden der Daten, die von einem oder mehreren Sensoren empfangen wurden, mit den Informationen, die für das geografische Informationssystem (GIS) spezifisch sind, um eine oder mehrere Kontextinformationen zu erhalten;
Vorverarbeiten der erhaltenen einen oder mehreren Kontextinformationen zusammen mit den Daten, die für den einen oder die mehreren Prozessparameter spezifisch sind, unter Verwendung einer oder mehrerer Datenvorverarbeitungstechniken;
Einspeisen der vorverarbeiteten Daten in ein oder mehrere trainierte Maschinenlernmodelle (ML-Modelle), wobei das eine oder die mehreren ML-Modelle unter Verwendung einer oder mehrerer ML-Trainingstechniken unter Verwendung von Daten aus einem Datensatz trainiert werden;
Vorhersagen einer Wahrscheinlichkeit von Escherichia coli (E. coli) unter Verwendung des trainierten einen oder der trainierten mehreren ML-Modelle;
Erhalten einer Korrelationsmatrix von einem oder mehreren Parametern, die in den vorverarbeiteten Daten enthalten sind, und der vorhergesagten Wahrscheinlichkeit von Escherichia coli (E. coli); und
Beurteilen von Kontaminationsrisiken, die mit der Wasserprobe assoziiert sind, basierend auf der erhaltenen Korrelationsmatrix.

6. System nach Anspruch 5, wobei der eine oder die mehreren Sensoren einen oder mehrere Temperatursensoren, einen oder mehrere Feuchtigkeitssensoren, einen oder mehrere pH-Sensoren, einen oder mehrere Sensoren für gelösten Sauerstoff, einen oder mehrere Trübungssensoren, einen oder mehrere Leitfähigkeitssensoren, einen oder mehrere Nährstoffsensoren, einen oder mehrere Durchflussmesser, einen oder mehrere Drucksensoren, einen oder mehrere Chlorsensoren, einen oder mehrere UV-Sensoren, einen oder mehrere chemische Sensoren, einen oder mehrere Biofilmsensoren, einen oder mehrere optische Sensoren und einen oder mehrere georäumliche Sensoren umfassen.

7. System nach Anspruch 5, wobei die Daten, die für das geografische Informationssystem (GIS) spezifisch sind, eine Höhe, einen oder mehrere Landnutzungstypen und eine oder mehrere Wasserquellennähen umfassen.

8. System nach Anspruch 5, wobei die Daten, die für einen oder mehrere Prozessparameter spezifisch sind, eine oder mehrere Durchflussraten, eine oder mehrere Retentionszeiten, eine Desinfektionswirksamkeit und eine oder mehrere Eigenschaften, die für Rohrmaterial, das Wasser trägt, spezifisch sind, umfassen.

9. Ein oder mehrere nichtflüchtige maschinenlesbare Informationsspeichermedien, die eine oder mehrere Anweisungen umfassen, die, wenn sie von einem oder mehreren Hardwareprozessoren ausgeführt werden, bewirken:
Empfangen (i) von Daten von einem oder mehreren Sensoren, die für eine Wasserprobe spezifisch sind, die aus Wasser gesammelt wurde, das in einem Rohr fließt und in einer Vorrichtung gespeichert wurde, (ii) von Informationen, die für ein geografisches Informationssystem (GIS) spezifisch sind, das sich auf eine Region bezieht, von der die Wasserprobe gesammelt wurde, und (iii) von Daten, die für einen oder mehrere Prozessparameter spezifisch sind, die sich auf einen Wasserspeicher und eine Verarbeitungseinheit beziehen;
Abbilden der Daten, die von einem oder mehreren Sensoren empfangen wurden, mit den Informationen, die für das geografische Informationssystem (GIS) spezifisch sind, um eine oder mehrere Kontextinformationen zu erhalten;
Vorverarbeiten der erhaltenen einen oder mehreren Kontextinformationen zusammen mit den Daten, die für den einen oder die mehreren Prozessparameter spezifisch sind, unter Verwendung einer oder mehrerer Datenvorverarbeitungstechniken;
Einspeisen der vorverarbeiteten Daten in ein oder mehrere trainierte Maschinenlernmodelle (ML-Modelle), wobei das eine oder die mehreren ML-Modelle unter Verwendung einer oder mehrerer ML-Trainingstechniken unter Verwendung von Daten aus einem Datensatz trainiert werden;
Vorhersagen einer Wahrscheinlichkeit von Escherichia coli (E. coli) unter Verwendung des trainierten einen oder der trainierten mehreren ML-Modelle;
Erhalten einer Korrelationsmatrix von einem oder mehreren Parametern, die in den vorverarbeiteten Daten enthalten sind, und der vorhergesagten Wahrscheinlichkeit von Escherichia coli (E. coli); und
Beurteilen von Kontaminationsrisiken, die mit der Wasserprobe assoziiert sind, basierend auf der erhaltenen Korrelationsmatrix.

10. Ein oder mehrere nichtflüchtige maschinenlesbare Informationsspeichermedien nach Anspruch 9, wobei der eine oder die mehreren Sensoren einen oder mehrere Temperatursensoren, einen oder mehrere Feuchtigkeitssensoren, einen oder mehrere pH-Sensoren, einen oder mehrere Sensoren für gelösten Sauerstoff, einen oder mehrere Trübungssensoren, einen oder mehrere Leitfähigkeitssensoren, einen oder mehrere Nährstoffsensoren, einen oder mehrere Durchflussmesser, einen oder mehrere Drucksensoren, einen oder mehrere Chlorsensoren, einen oder mehrere UV-Sensoren, einen oder mehrere chemische Sensoren, einen oder mehrere Biofilmsensoren, einen oder mehrere optische Sensoren und einen oder mehrere georäumliche Sensoren umfassen.

11. Ein oder mehrere nichtflüchtige maschinenlesbare Informationsspeichermedien nach Anspruch 9, wobei die Daten, die für das GIS spezifisch sind, eine Höhe, einen oder mehrere Landnutzungstypen und eine oder mehrere Wasserquellennähen umfassen.

12. Ein oder mehrere nichtflüchtige maschinenlesbare Informationsspeichermedien nach Anspruch 9, wobei die Daten, die für einen oder mehrere Prozessparameter spezifisch sind, eine oder mehrere Durchflussraten, eine oder mehrere Retentionszeiten, eine Desinfektionswirksamkeit und eine oder mehrere Eigenschaften, die für Rohrmaterial, das Wasser trägt, spezifisch sind, umfassen.

## Revendications

1. Procédé mis en oeuvre par processeur (300), comprenant :
la réception (302), par l'intermédiaire d'un ou plusieurs processeurs matériels, (i) de données provenant d'un ou plusieurs capteurs spécifiques à un échantillon d'eau collecté à partir d'eau s'écoulant dans un tuyau et stocké dans un appareil, (ii) d'informations spécifiques à un système d'information géographique (GIS) concernant une région à partir de laquelle l'échantillon d'eau est collecté et (iii) de données spécifiques à un ou plusieurs paramètres de processus concernant un stockage d'eau et une unité de traitement ;
la mise en correspondance (304), par l'intermédiaire des un ou plusieurs processeurs matériels, des données reçues à partir d'un ou plusieurs capteurs avec les informations spécifiques au système d'information géographique (GIS) pour obtenir une ou plusieurs informations contextuelles ;
le prétraitement (306), par l'intermédiaire des un ou plusieurs processeurs matériels, des une ou plusieurs informations contextuelles obtenues conjointement avec les données spécifiques aux un ou plusieurs paramètres de processus en utilisant une ou plusieurs techniques de prétraitement de données ;
l'alimentation (308), par l'intermédiaire des un ou plusieurs processeurs matériels, des données prétraitées dans un ou plusieurs modèles d'apprentissage machine (ML) entraînés, dans lequel les un ou plusieurs modèles ML sont entraînés en utilisant une ou plusieurs techniques d'entraînement ML en utilisant des données provenant d'un ensemble de données ;
la prédiction (310), par l'intermédiaire des un ou plusieurs processeurs matériels, d'une probabilité d'Escherichia coli (E. coli) en utilisant les un ou plusieurs modèles ML entraînés ;
l'obtention (312), par l'intermédiaire des un ou plusieurs processeurs matériels, d'une matrice de corrélation d'un ou plusieurs paramètres compris dans les données prétraitées et la probabilité prédite d'Escherichia coli (E. coli) ; et
l'évaluation (314), par l'intermédiaire des un ou plusieurs processeurs matériels, de risques de contamination associés à l'échantillon d'eau sur la base de la matrice de corrélation obtenue.

2. Procédé mis en oeuvre par processeur selon la revendication 1, dans lequel les un ou plusieurs capteurs comprennent un ou plusieurs capteurs de température, un ou plusieurs capteurs d'humidité, un ou plusieurs capteurs de pH, un ou plusieurs capteurs d'oxygène dissous, un ou plusieurs capteurs de turbidité, un ou plusieurs capteurs de conductivité, un ou plusieurs capteurs de nutriments, un ou plusieurs débitmètres, un ou plusieurs capteurs de pression, un ou plusieurs capteurs de chlore, un ou plusieurs capteurs UV, un ou plusieurs capteurs chimiques, un ou plusieurs capteurs de biofilm, un ou plusieurs capteurs optiques et un ou plusieurs capteurs géospatiaux.

3. Procédé mis en oeuvre par processeur selon la revendication 1, dans lequel les données spécifiques au GIS comprennent une altitude, un ou plusieurs types d'utilisation du sol et une ou plusieurs proximités de source d'eau.

4. Procédé mis en oeuvre par processeur selon la revendication 1, dans lequel les données spécifiques à un ou plusieurs paramètres de processus comprennent un ou plusieurs débits, un ou plusieurs temps de rétention, une efficacité de désinfection, et une ou plusieurs caractéristiques spécifiques à un matériau de tuyau transportant de l'eau.

5. Système (100), comprenant :
une mémoire (104) stockant des instructions ;
une ou plusieurs interfaces de communication (112) ; et
un ou plusieurs processeurs matériels (102) couplés à la mémoire (104) par l'intermédiaire des une ou plusieurs interfaces de communication (112), dans lequel les un ou plusieurs processeurs matériels (102) sont configurés par les instructions pour :
recevoir (i) des données provenant d'un ou plusieurs capteurs spécifiques à un échantillon d'eau collecté à partir d'eau s'écoulant dans un tuyau et stocké dans un appareil, (ii) des informations spécifiques à un système d'information géographique (GIS) concernant une région à partir de laquelle l'échantillon d'eau est collecté et (iii) des données spécifiques à un ou plusieurs paramètres de processus concernant un stockage d'eau et une unité de traitement ;
mettre en correspondance les données reçues à partir d'un ou plusieurs capteurs avec les informations spécifiques au système d'information géographique (GIS) pour obtenir une ou plusieurs informations contextuelles ;
prétraiter les une ou plusieurs informations contextuelles obtenues conjointement avec les données spécifiques aux un ou plusieurs paramètres de processus en utilisant une ou plusieurs techniques de prétraitement de données ;
alimenter les données prétraitées dans un ou plusieurs modèles d'apprentissage machine (ML) entraînés, dans lequel les un ou plusieurs modèles ML sont entraînés en utilisant une ou plusieurs techniques d'entraînement ML en utilisant des données provenant d'un ensemble de données ;
prédire une probabilité d'Escherichia coli (E. coli) en utilisant les un ou plusieurs modèles ML entraînés ;
obtenir une matrice de corrélation d'un ou plusieurs paramètres compris dans les données prétraitées et la probabilité prédite d'Escherichia coli (E. coli) ; et
évaluer des risques de contamination associés à l'échantillon d'eau sur la base de la matrice de corrélation obtenue.

6. Système selon la revendication 5, dans lequel les un ou plusieurs capteurs comprennent un ou plusieurs capteurs de température, un ou plusieurs capteurs d'humidité, un ou plusieurs capteurs de pH, un ou plusieurs capteurs d'oxygène dissous, un ou plusieurs capteurs de turbidité, un ou plusieurs capteurs de conductivité, un ou plusieurs capteurs de nutriments, un ou plusieurs débitmètres, un ou plusieurs capteurs de pression, un ou plusieurs capteurs de chlore, un ou plusieurs capteurs UV, un ou plusieurs capteurs chimiques, un ou plusieurs capteurs de biofilm, un ou plusieurs capteurs optiques et un ou plusieurs capteurs géospatiaux.

7. Système selon la revendication 5, dans lequel les données spécifiques au système d'information géographique (GIS) comprennent une altitude, un ou plusieurs types d'utilisation du sol et une ou plusieurs proximités de source d'eau.

8. Système selon la revendication 5, dans lequel les données spécifiques à un ou plusieurs paramètres de processus comprennent un ou plusieurs débits, un ou plusieurs temps de rétention, une efficacité de désinfection, et une ou plusieurs caractéristiques spécifiques à un matériau de tuyau transportant de l'eau.

9. Un ou plusieurs supports de stockage d'informations lisibles par machine non transitoires comprenant une ou plusieurs instructions qui, lorsqu'elles sont exécutées par un ou plusieurs processeurs matériels, amènent :
la réception (i) de données provenant d'un ou plusieurs capteurs spécifiques à un échantillon d'eau collecté à partir d'eau s'écoulant dans un tuyau et stocké dans un appareil, (ii) d'informations spécifiques à un système d'information géographique (GIS) concernant une région à partir de laquelle l'échantillon d'eau est collecté et (iii) de données spécifiques à un ou plusieurs paramètres de processus concernant un stockage d'eau et une unité de traitement ;
la mise en correspondance des données reçues à partir d'un ou plusieurs capteurs avec les informations spécifiques au système d'information géographique (GIS) pour obtenir une ou plusieurs informations contextuelles ;
le prétraitement des une ou plusieurs informations contextuelles obtenues conjointement avec les données spécifiques aux un ou plusieurs paramètres de processus en utilisant une ou plusieurs techniques de prétraitement de données ;
l'alimentation des données prétraitées dans un ou plusieurs modèles d'apprentissage machine (ML) entraînés, dans lequel les un ou plusieurs modèles ML sont entraînés en utilisant une ou plusieurs techniques d'entraînement ML en utilisant des données provenant d'un ensemble de données ;
la prédiction d'une probabilité d'Escherichia coli (E. coli) en utilisant les un ou plusieurs modèles ML entraînés ;
l'obtention d'une matrice de corrélation d'un ou plusieurs paramètres compris dans les données prétraitées et la probabilité prédite d'Escherichia coli (E. coli) ; et
l'évaluation de risques de contamination associés à l'échantillon d'eau sur la base de la matrice de corrélation obtenue.

10. Un ou plusieurs supports de stockage d'informations lisibles par machine non transitoires selon la revendication 9, dans lequel les un ou plusieurs capteurs comprennent un ou plusieurs capteurs de température, un ou plusieurs capteurs d'humidité, un ou plusieurs capteurs de pH, un ou plusieurs capteurs d'oxygène dissous, un ou plusieurs capteurs de turbidité, un ou plusieurs capteurs de conductivité, un ou plusieurs capteurs de nutriments, un ou plusieurs débitmètres, un ou plusieurs capteurs de pression, un ou plusieurs capteurs de chlore, un ou plusieurs capteurs UV, un ou plusieurs capteurs chimiques, un ou plusieurs capteurs de biofilm, un ou plusieurs capteurs optiques et un ou plusieurs capteurs géospatiaux.

11. Un ou plusieurs supports de stockage d'informations lisibles par machine non transitoires selon la revendication 9, dans lequel les données spécifiques au GIS comprennent une altitude, un ou plusieurs types d'utilisation du sol et une ou plusieurs proximités de source d'eau.

12. Un ou plusieurs supports de stockage d'informations lisibles par machine non transitoires selon la revendication 9, dans lequel les données spécifiques à un ou plusieurs paramètres de processus comprennent un ou plusieurs débits, un ou plusieurs temps de rétention, une efficacité de désinfection, et une ou plusieurs caractéristiques spécifiques à un matériau de tuyau transportant de l'eau.
